Europäisches Patentamt

European Patent Office

Office européen des brevets

(11) Publication number: **0 316 495**

**A1**

(12) **EUROPEAN PATENT APPLICATION**

(21) Application number: 87402621.4

(51) Int. Cl.⁴: **C07K 7/08 , G01N 33/569**

(22) Date of filing: **19.11.87**

(43) Date of publication of application:
24.05.89 Bulletin 89/21

(84) Designated Contracting States:
AT BE CH DE FR GB IT LI NL SE

(71) Applicant: **IAF BIOCHEM INTERNATIONAL INC.**
**531, Des Prairies Boulevard**
**Laval Quebec H7V 1B7(CA)**

(72) Inventor: **Bellini, Francesco**
**3045, Graham Boulevard Town of Mount**
**Royal**
**Quebec H3R 1J8(CA)**
Inventor: **Dionne, Gervais**
**1735, O'Brien Street**
**St-Laurent Quebec H4L 3W5(CA)**
Inventor: **Lacroix, Martial**
**1925 Croissant Tourangereau**
**Brossard Quebec J4W 3H8(CA)**

(74) Representative: **Ayache, Monique et al**
**CABINET PLASSERAUD 84, rue d'Amsterdam**
**F-75009 Paris(FR)**

(54) **Synthetic peptides useful for the detection of aids-related antibodies in human sera.**

(57) Compounds of the formula:
$H_2N$-Ser-Gly-Lys-Leu-Ile-Cys-Thr-Thr-Ala-Val-Pro-Trp-Asn-Ala-Ser-COOH
and
$H_2N$-Tyr-Ser-Gly-Lys-Leu-Ile-Cys-Thr-Thr-Ala-Val-Pro-Trp-Asn-Ala-Ser-COOH
when coupled to a proper carrier are useful for the detection of LAV/HTLV-III antibodies in human sera and are used for the development of a diagnostic test for AIDS. These compounds provide for an efficient, precise and cheap routine diagnostic test of the above antibodies.

EP 0 316 495 A1

# SYNTHETIC PEPTIDES USEFUL FOR THE DETECTION OF AIDS-RELATED ANTIBODIES IN HUMAN SERA

## BACKGROUND OF THE INVENTION

### 1. Field of the Invention

The present invention relates to novel synthetic peptides useful for the detection of acquired Immunodeficiency Syndrome (AIDS) - related antibodies in human sera and to the method of preparing these peptides and peptide-carrier conjugates. The invention also relates to a diagnostic test for the detection of these antibodies. More particularly, the invention is directed to compounds of the formula

$H_2N$-Ser-Gly-Lys-Leu-Ile-Cys-Thr-Thr-Ala-Val-Pro-Trp-Asn-Ala-Ser-COOH
and
$H_2N$-Tyr-Ser-Gly-Lys-Leu-Ile-Cys-Thr-Thr-Ala-Val-Pro-Trp-Asn-Ala-Ser-COOH

and to peptide-carrier conjugates which are useful for the detection of LAV/HTLV-III antibodies in human sera. The invention is also concerned with a method for the detection of these antibodies by means of a diagnostic test involving the use of the above peptide-carrier conjugates.

### 2. Description of the Prior Art

Human T Lymphotropic Virus-III/Lymphotropic Associated Virus (HTLV-III/LAV) is considered to be the primary cause of the Acquired Immunodeficiency Syndrome (AIDS).

Most patients infected by HTLV-III/LAV develop antiviral antibodies against the major viral proteins: the core protein p25 and the 2 envelope-glycoproteins gp41 and gp110.

Existing serological tests for the diagnosis of AIDS are based on the detection of antibodies directed against the virus since the presence of serum antibodies indicates the presence of virus. The currently available serological tests for AIDS include the ELISA technique using purified virus or biosynthetic viral peptides. Western blotting or radio immunoprecipitation assay, using viral particle preparations and indirect immunofluorescence using virus-infected cells are examples of presently available tests.

The major problem encountered by using the ELISA technique is the high degree of false positive results due to non specific reaction of patients serum with the antigenic material used in the diagnostic kit. Such non specific reaction will be eliminated if synthetic peptides, specific of the virus, are to be used as tests antigens in serological assays.

It has been shown by Chang et al., Biotechnology 3, 905-909 (1985) that a peptide containing 82 amino acid residues, encoded by a gene segment in the env-lor region of HTLV-III, produced by E. coli. can reproducibly detect antibodies in sera of patients with HTLV-III infections. Earlier, Sela, Adv. Immunol. 5, 29 [1966] had shown that in a protein, an antigenic site can take the size of a peptide with 6 to 7 amino acid residues.

## SUMMARY OF INVENTION

It is an object of the present invention to provide novel peptides which when conjugated to a proper carrier can detect anti-LAV/HTLV-III antibodies in sera taken from patients with AIDS.

It is another object of the present invention to provide a convenient process for preparing the novel peptides according to the invention, which process starts from readily available materials, avoid noxious reagents, is executed smoothly and utilises easily removable protecting groups.

It is another object of the present invention to provide peptide-carrier conjugates which are useful for the detection of anti-LAV/HTLV-III antibodies in sera samples.

It is another object of the present invention to provide a diagnostic test utilizing the above peptide-carrier conjugates.

In accordance with the invention, there are provided synthetic peptides of the formula:

$H_2N$-Ser-Gly-Lys-Leu-Ile-Cys-Thr-Thr-Ala-Val-Pro-Trp-Asn-Ala-Ser-COOH
and
$H_2N$-Tyr-Ser-Gly-Lys-Leu-Ile-Cys-Thr-Thr-Ala-Val-Pro-Trp-Asn-Ala-Ser-COOH

According to the invention, there are provided conjugates derived from the above peptides and a suitable carrier, such as keyhole limpet hemocyanin (KLH).

According to the invention, there is also provided a method of preparing the above peptides by providing a resin support, coupling amino and hydroxy-protected serine to the resin support, removing the amino protecting group from the coupled product, and sequentially coupling $\alpha$-amino protected amino acids to produce the synthetic peptides.

According to the invention, there is also provided a method for the detection of anti-LAV/HTLV-III antibodies in serum samples which comprises providing a given quantity of the above peptide-carrier conjugate, adding the conjugate to a serum sample which may contain the above antibodies, and detecting the presence or absence of these antibodies in the serum sample.

## DESCRIPTION OF PREFERRED EMBODIMENTS

The first synthetic peptide has an amino acid sequence which duplicates the sequence of the env-lor region 606-620 of HTLV-III and it corresponds to the amino acid sequence 40-54 of the "peptide 121" of Chang et al., Biotechnology 3, 905-909 (1985). The second peptide is an analog of the env-lor region 605-620 of HTLV-III in which the cysteine 605 has been substituted by a tyrosine.

These compounds when conjugated to a proper carrier (BSA, KLH, poly-Ala-Lys) can be used to detect anti-LAV/HTLV-III antibodies in sera from patients with AIDS or AIDS-related complex (ARC). In the use aspect of this invention, the new compounds provide for an efficient, precise and cheap routine diagnostic test of anti-LAV/HTLV-III antibodies.

The peptides of this invention are prepared by solid phase methodology, following techniques generally known in the art for building an amino acid sequence from an initial resin supported amino acid. The products may be prepared according to the technique of Merrifield J. Am. Chem. Soc. 85, 2141 (1963). However the modifications of Chang and Meinehofer, Int. J. Peptide Protein Res. 11, 246 (1978) generally illustrate the preferred techniques used in the present invention.

The resin support employed may be any suitable resin conventionally employed in the art for solid phase preparation of polypeptides, preferably polystyrene which has been crosslinked with 0.5 to about 3 percent divinyl benzene which has been chloromethylated to provide sites for ester formation with the initially introduced protected amino acid or preferably by using a more acid labile linkage by the introduction of the first protected amino acid on a p-benzyloxyalcohol resin following the method of Meinehofer et al., Int. J. Peptide Protein Res. 13, 35 (1979). This resin support is suitably prepared by reacting the chloromethylated resin with 4-hydroxymethyl phenol according to Lu et al., J. Org. Chem. 46, 3433 (1981). Following the coupling of the amino and hydroxy protected serine to the resin support, the amino protecting group is removed by standard methods conventionally employed in the art of solid phase peptide synthesis: namely by using dilute trifluoroacetic acid in methylene chloride or piperidine in dimethylformamide. After removal of the amino protecting group the remaining $\alpha$-amino protected and, if necessary, side chain protected amino acids are coupled, sequentially, in the desired order to obtain the product. Alternatively, multiple amino acid groups may be coupled using solution methodology prior to coupling with the resin-supported amino acid sequence. The selection of an appropriate coupling reagent follows established art. For instance, suitable coupling reagents are N,N'-diisopropylcarbodiimide or N,N'-dicyclohexylcarbodiimide (DCC) either alone or preferably in the presence of 1-hydroxybenzotriazole. Another useful coupling procedure makes use of preformed symmetrical anhydrides of protected amino acids.

Each protected amino acid or amino acid sequence is introduced into the solid phase reactor in a two to six fold molar excess and the coupling is carried out in a medium of dimethylformamide: methylene chloride or in either dimethylformamide or methylene chloride alone. In cases where incomplete coupling occurs, the coupling procedure is repeated before removal of the $\alpha$-amino protecting group, prior to introduction of the next amino acid to the solid phase reactor. The extent of the coupling reaction at each stage of the synthesis is monitored by the ninhydrin reaction as described by E. Kaiser et al., Anal. Biochem., 34, 595 (1970).

The necessary $\alpha$-amino protecting group employed for each amino acid introduced onto the growing polypeptide chain is preferably 9-fluorenylmethyloxycarbonyl (Fmoc), although any other suitable protecting group may be employed as long as it does not suffer degradation under the coupling conditions while being readily removable selectively in the presence of any other protecting groups already present in the growing molecule. Additional examples of such alternative $\alpha$-amino protecting groups, after taking into consideration of the structural features of the rest of the polypeptide molecule, are tert-butyloxycarbonyl (Boc), 2-(4-biphenyl)propyl-2-oxycarbonyl (Bpoc), or 2(3,5 dimethoxyphenyl)propyl-2-oxycarbonyl (Ddz) and related reagents.

The criteria for selecting protecting groups for the side chain amino acids are (a) stability of the protecting group to the various reagent under reaction conditions selective for the removal of the $\alpha$-amino protecting group at each step of the synthesis: (b) the protecting group must retain its strategic properties (i.e. not be split off under coupling conditions) and (c) the protecting group must be readily removable upon conclusion of the polypeptide synthesis and under conditions that do not otherwise effect the polypeptide structure. The preferred amino acid side-chain protecting groups used in the invention are tert-butyl for serine, threonine and tyrosine, tert-butyloxycarbonyl (Boc) for lysine and trityl for cysteine.

The fully protected resin-supported peptides are cleaved from p-benzyloxy alcohol resin with a 50 to 60 percent solution of trifluoroacetic acid in methylene chloride over 1 to 6 hours at room temperature in the presence of appropriate scavengers such as anisole, thioanisole, ethyl methyl sulfide, 1,2-ethanedithiol and related reagents. Simultaneously, all acid labile side-chain protecting groups are removed.

In order to raise antibodies and to perform immunoassays, the purified peptides are coupled to any suitable carrier such as thyroglobulin, bovine serum albumin (BSA), ovalbumin, keyhole limpet haemocyanin (KLH) or poly-DL-alanine-poly-L-lysine and related molecules. The selection of the conjugation methods for the generation of the peptide-carrier conjugate follows established art. The preferred coupling method for reagent attachment is a water soluble bifunctional sulfo-maleimidobenzoyl-N-hydroxysuccinimide ester. In the first step, the amino groups of the keyhole limpet haemocyanin (KLH) are acylated by the activated N-hydroxysuccinimide ester group of the reagent to yield a KLH carrying a space-out maleimide moiety. In the second step, the latter is reacted with the sulfhydryl group of the synthetic peptide to give the peptide-carrier conjugate.

## EXAMPLES

### EXAMPLE 1

Preparation of resin carrying 9-fluorenylmethyloxycarbonyl-O-tert-butyl serine residues

9-fluorenylmethyloxycarbonyl-O-tert-butyl serine (4.02 g, 10.5 mmoles) in methylene chloride (40 mL)-dimethylformamide (10 mL) mixture was added to a suspension of 20 g of p-benzyloxy alcohol resin (substitution 0.35 meq./g, 7.0 mmoles) in methylene chloride (160 mL)-dimethylformamide (40 mL) mixture at $0°C$. The mixture was stirred by hand for a few seconds and then treated with N,N'-dicyclohexylcarbodiimide (DCC) (2.38 g, 11.55 mmoles) followed by a catalytic amount of 4-dimethylaminopyridine (128 mg, 1.05 mmole). The mixture was stirred at $0°C$ for an additional 30 minutes and then at room temperature overnight. The filtered resin was washed successively with methylene chloride, dimethylformamide and isopropanol (3 washes for each) and finally with methylene chloride. The resin was suspended in methylene chloride (200 mL) chilled in an ice bath and to the suspension was added with stirring redistilled pyridine (3.6 mL, 45 mmoles) followed by benzoyl chloride (5.2 mL, 45 mmoles). Stirring was continued at $0°C$ for 30 minutes and then at room temperature for 60 minutes After filtration, the resin was washed successively with methylene chloride, dimethylformamide and isopropanol (3 washes each) and finally with petroleum ether (twice) and dried under high vacuum to a constant weight of 23.8 g.

Spectrophotometric determination of substitution according to Meinehofer et al., Int. J. Peptide Protein Res. 13, 35 (1979) indicated a substitution on the resin amounting to 0.325 meq./g.

### EXAMPLE 2

O-tert-butyl-L-seryl-glycyl-N-tert-butyloxy-carbonyl-L-lysyl-L-leucyl-L-isoleucyl-S-trityl-L-cysteinyl-O-tert-butyl-L-threonyl-O-tert-butyl-L-threonyl-L-alanyl-L-valinyl-L-prolyl-L-tryptophyl-L-asparaginyl-L-alanyl-O-tert-butyl-seryl-acyl resin ester

$$\overset{\overset{\displaystyle OBu^t}{|}}{H_2N-Ser}-Gly-\overset{\overset{\displaystyle N^\epsilon Boc}{|}}{Lys}-Leu-Ile-\overset{\overset{\displaystyle STrt}{|}}{Cys}-\overset{\overset{\displaystyle OBu^t}{|}}{Thr}-\overset{\overset{\displaystyle OBu^t}{|}}{Thr}-Ala-Val-Pro-Trp-Asn-$$

$$\overset{\overset{\displaystyle OBu^t}{|}}{Ala-Ser}-O-Resin$$

The preceding 9-fluorenylmethyloxycarbonyl-O-tert-butyl-serine resin (4.0 g) from Example 1 was placed in a 125 mL reaction vessel of a Labortec SP 640 peptide synthesizer and treated as follows:

1. Wash with 50 mL of dimethylformamide (twice for one minute each)

2. Prewash with 60 mL of a 20 percent solution of piperidine in dimethylformamide (3 min.)
Deprotection with 60 mL of a 20 percent solution of piperidine in dimethylformamide (10 min.)

4. Wash with 50 mL of dimethylformamide (4 times for 30 seconds each)

5. Wash with 60 mL of isopropanol (twice for 30 seconds each)

6. Wash with 50 mL of dimethylformamide (twice for 45 seconds each)

7. Check for free amine groups - Kaiser test (which must be positive)

8. The peptide resin is then gently shaken for 2 minutes with 3 molar equivalents of the desired Fmoc-protected amino acid and 3.6 molar equivalents of 1-hydroxybenzotriazole all dissolved in 60 mL of dry redistilled dimethylformamide

9. Solid N,N'-dicyclohexylcarbodiimide (DCC) (3.3 molar equivalents) is then added to the reaction vessel

10. The reaction mixture is shaken for 2 hours

11. Wash with 60 mL of dimethylformamide (twice for 45 seconds each)

12. Wash with 60 mL of isopropanol (twice for 45 seconds each).

After step 12, an aliquot is taken for a ninhydrin test: when the test is negative, one goes back to step 1 for coupling of the next amino acid; if the test is positive or slightly positive, one goes back to step 6 and proceeds up to 12 again.

The above schedule is used for coupling of each of the amino acids of the peptides described in the invention. N-$\alpha$-protection with 9-fluorenylmethyloxycarbonyl (Fmoc) is used for each of the remaining amino acids throughout the synthesis.

The following amino acid residues were then introduced consecutively: Fmoc-L-alanine (4 mmoles), Fmoc-L-asparagine (4 mmoles), Fmoc-L-tryptophane (4 mmoles), Fmoc-L-proline (4 mmoles), Fmoc-L-valine (4 mmoles), Fmoc-L-alanine (4 mmoles), Fmoc-O-tert-butyl-L-threonine (4 mmoles), Fmoc-O-tert-butyl-L-threonine (4 mmoles), Fmoc-S-trityl-L-cysteine (4-mmoles), Fmoc-L-isoleucine (4 mmoles), Fmoc-L-leucine (4 mmoles), Fmoc-N$^\epsilon$-tert-butyloxycarbonyl-L-lysine (4 mmoles), Fmoc-glycine (4 mmoles) and Fmoc-O-tert-butyl-L-serine (4 mmoles). After the addition of the last amino acid, the N-$\alpha$-Fmoc group of the N-terminal serine residue is removed by going back to steps 1 through 7 of the above schedule. The peptide resin was washed with methylene chloride and dried in vacuo to give 6.8 g of solid material.

EXAMPLE 3

L-seryl-glycyl-L-lysyl-L-leucyl-L-isoleucyl-L-cysteinyl-L-threonyl-L-threonyl-L-alanyl-L-valinyl-L-prolyl-L-tryptophyl-L-asparaginyl-L-alanyl-L-serine

$H_2N$-Ser-Gly-Lys-Leu-Ile-Cys-Thr-Thr-Ala-Val-Pro-Trp-Asn-Ala-Ser-COOH

The protected peptide-resin (3.4 g) from above (Example 2) was suspended in 30 mL of a 55 percent solution of trifluoroacetic acid (TFA) in methylene chloride containing 2.5 percent 1,2-ethanedithiol and 2.5 percent of anisole. The mixture was flushed with nitrogen and stirred for 1.5 hour at room temperature. The mixture was filtered and the resin washed with methylene chloride. The resin was treated again with 30 mL of a 20 percent solution of trifluoroacetic acid (TFA) in methylene chloride containing 1,2-ethanedithiol (0.25%) for 5 minutes at room temperature. The mixture was filtered and the resin washed with a 20 percent solution of TFA in methylene chloride and then with methylene chloride. The combined filtrates were evaporated in vacuo below 35°C and the residue triturated several times with dried diethyl ether. The solid was dissolved in 10% aqueous acetic acid and lyophylized to afford the crude title product (667 mg).

## EXAMPLE 4

Purification and characterization of L-seryl-glycyl-L-lysyl-L-leucyl-L-isoleucyl-L-cysteinyl-L-threonyl-L-threonyl-L-alanyl-L-valinyl-L-prolyl-L-tryptophyl-L-asparaginyl-L-alanyl-L-serine

The above crude peptide (Example 3) (410 mg) was purified by preparative HPLC on a column (2.5 x 25 cm) of Vydac $C_{18}$ silica (10 $\mu$ particle size, 300 Å pores size) and the product eluted with a linear gradient ranging from 15% of solvent A to 55% of solvent B over 45 minutes at a flow rate of 8 mL/minute (solvent A is 0.1 M triethylammonium phosphate pH 2.25 and solvent B is a mixture of acetonitrile (60%) and A (40%)). The effluent was monitored at 220 nm and the fractions examined by analytical HPLC under the conditions described below and the relevant pure fractions pooled. The acetonitrile of the pooled fractions was evaporated at room temperature under vacuum and the remaining solution desalted on the same column using a linear gradient ranging from 0.06% trifluoroacetic acid in water to 0.06% trifluoroacetic acid in acetonitrile (0% to 60% in 20 minutes). The pooled fractions were then lyophylized to yield 177 mg of the purified product.

This purified material was examined for homogeneity by analytical HPLC on a Varian 5000 system using a $C_{18}$ $\mu$-Bondapak column (3.9 mm x 30 cm) using a linear gradient ranging from 0.06% TFA in water to 0.06% TFA in acetonitrile (0 to 50% in 50 minutes) at a flow rate of 1 mL per minute and at a pressure of 62 atm. Peaks were detected at 220 nm using a Varian variable-wavelength detector. The desired product eluted with a retention time of 35.9 min and was homogeneous to 96%.

The RF value of the peptide (30 $\mu$g load) in the thin layer assay system (silica, Merck F254) n-butanol: acetic acid: ethyl acetate: water (1:1:1:1) was 0.39. With formic acid: acetic acid: water: methanol (1:7:2:10) as solvent, the Rf was 0.72.

Hydrolysis of the peptide in 6N hydrochloric acid (0.2 mL/20 nmoles peptide) for 20 hours at 110°C in a closed evacuated system revealed the presence of all the required amino acids in the following ratios: Lys 1.03(1), Trp 1.15(1), Asp 0.99(1), Thr 1.84(2), Ser 1.81(2), Pro 1.02(1), Gly 0.99(1), Ala 1.99(2), ½Cys 0.5(1), Val 0.99(1), Ile 0.73(1), Leu 1.0(1).

The desired peptide was determined to constitute 94% of the purified solids.

## EXAMPLE 5

L-tyrosyl-L-seryl-glycyl-L-lysyl-L-leucyl-L-isoleucyl-L-cysteinyl-L-threonyl-L-threonyl-L-alanyl-L-valinyl-L-prolyl-L-tyrptophyl-L-asparaginyl-L-alanyl-L-serine

$H_2$N-Tyr-Ser-Gly-Lys-Leu-Ile-Cys-Thr-Thr-Ala-Val-Pro-Trp-Asn-Ala-Ser-OH

680 mg of the peptide resin of Example 2 was reacted with Fmoc-O-tert-butyl-L-tyrosine according to the procedure described in Example 2. The Fmoc group was removed, the peptide resin was washed with methylene chloride and dried in vacuo to yield 760 mg of solids. This peptide resin was submitted to cleavage and deprotection step using exactly the same procedure described in Example 3 to give the crude title compound (150 mg). The latter was purified as in Example 4 to give 37.5 mg of the pure title compound.

The purified material was examined for homogeneity by analytical HPLC as described in Example 4. The product eluted with a retention time of 37.2 min. and was homogeneous to 97%.

The RF value of the peptide (30 μg load) in thin layer assay systems (silica, Merck F254) n-butanol: acetic acid: ethyl acetate: water (1:1:1:1) was 0.61. In the solvent chloroform: methanol: 32% acetic acid (5:3:1) the Rf was 0.56.

Hydrolysis of the peptide in 6N hydrochloric acid (0.2 mL/20 nmoles peptide) for 20 hours at 110° C in a closed evacuated system revealed the presence of all the required amino acids in the following ratios: Lys 1.0(1), Trp 1.13(1), Asp 0.97(1), Thr 1.82(2), Ser 1.80(2), Pro 1.0(1), Gly 1.0(1), Ala 1.94(2), ½ Cys 0.72(1), Val 0.96(1), Ile 0.72(1), Leu 1.0(1), Tyr 0.97(1).

The peptide purity amounted to 94% as determined.

EXAMPLE 6

Coupling of L-seryl-glycyl-L-lysyl-L-leucyl-L-isoleucyl-L-cysteinyl-L-threonyl-L-threonyl-L-alanyl-L-valinyl-L-prolyl-L-tryptophyl-L-asparaginyl-L-alanyl-L-serine to keyhole limpet hemocyanin (KLH)

A solution of sulfosuccinimidyl-4-(p-maleimidophenyl butyrate) (sulfo-SMPB, Pierce Chemical) (13.9 mg) in water (1.4 ml) was added to a solution of keyhole limpet hemocyanin (KLH) (17.9 mg) in 0.9 ml of 0.02 M sodium phosphate buffer (pH 7.0). The resulting solution was shaken at room temperature for 45 minutes and the activated carrier immediately applied to a Sephadex G-25TM column equilibrated with 0.1 M sodium phosphate buffer (pH 6.0) at 4° C. Elution with this buffer removed unreacted sulfo-SMPB. The fractions corresponding to the first peak of absorbance at 280 nm were pooled and contained the activated KLH. This pool was transferred into a round bottom flask and a solution of the title peptide (20 mg) in 2.0 ml of 0.05 M sodium phosphate buffer (pH 6.2) was added. The mixture was thoroughly flushed with nitrogen and incubated overnight at room temperature. It was then dialysed against a phosphate-buffered solution (pH 7.4). The coupling efficiency was monitored using radioactively labeled peptide and found to amount to 46%.

EXAMPLE 7

This example relates to the use of one of the above described peptides in a test to detect anti-LAV/HTLV-III antibodies in sera. Use was made of a plate binding assay performed according to the procedure of Engvall et al., J. Immunol., 109, 129-135 (1972) and Van Weeman et al., FEBS Letters 24, 77-81 (1972). Individuals rows of an ELISA plates were sensitized with either KLH, KLH-coupled peptide or with buffer only (Tris-HCl 0.01 M: NaCl 0.5M: Tween 20 0.05%: pH 7.5). In this example, a reference panel of 120 serum samples obtained from Dr. M. O'Shaughnessy (LCDC, Ottawa) was used.

A positive reaction indicative of the presence of anti-LAV/HTLV-III antibodies was observed in 51 out of 57 serum samples known to contain such antibodies. A negative reaction (absence of anti-LAV/HTLV-III antibodies) was observed in 63 out of 63 serum samples known to lack the presence of anti-LAV/HTLV-III antibodies. No false positive reaction was observed.

| | No. observed/No. tested | % |
|---|---|---|
| Positive | 51/57 | 89.5 |
| False negative | 6/57 | 10.5 |
| Negative | 63/63 | 100 |
| False positive | 0/63 | 0 |

**Claims**

1. Synthetic peptides selected from the group consisting of compounds of the formula:
H₂N-Ser-Gly-Lys-Leu-Ile-Cys-Thr-Thr-Ala-Val-Pro-Trp-Asn-Ala-Ser-COOH
and
H₂N-Tyr-Ser-Gly-Lys-Leu-Ile-Cys-Thr-Thr-Ala-Val-Pro-Trp-Asn-Ala-Ser-COOH.

2. Peptide-carrier conjugates resulting from the coupling of peptides according to claim 1 with a carrier capable of producing said conjugates.

3. Peptide-carrier conjugates according to claim 2, wherein said peptides are coupled to said carrier via sulfhydryl group.

4. Peptide-carrier conjugates according to claim 3, wherein said carried comprises keyhole limpet hemocyanin.

5. Method for the detection of anti-LAV/HTLV-III antibodies in serum samples, which comprises providing a given quantity of a peptide-carrier conjugate according to claim 4, adding said conjugate to a serum sample which may contain said antibodies and detecting the presence or absence of said antibodies in said serum sample.

# EUROPEAN SEARCH REPORT

European Patent Office

European Patent Office

Application Number

EP 87 40 2621

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | EP-A-0 227 169 (AKZO)<br>* Whole document * | 1,5 | C 07 K 7/08<br>G 01 N 33/569 |
| Y | US-A-4 629 783 (COSAND)<br>* Whole document * | 1,5 | |
| A | CHEMICAL ABSTRACTS, vol. 107, 1987, page 590, abstract no. 234358c, Columbus, Ohio, US; J.W. GNANN, Jr. et al.: "Diagnosis of AIDS by using a 12-amino acid peptide representing an immunodominant epitope of the human immunodeficiency virus", & J. INFECT. DIS 1987, 156(2), 261-7<br>* Abstract * | 1 | |
| A | CHEMICAL ABSTRACTS, vol. 108, 1988, page 443, abstract no. 20148f, Columbus, Ohio, US; J.W. GNANN, Jr. et al.: "Synthetic peptide immunoassay distinguishes HIV type 1 and HIV type 2 infections", & SCIENCE (WASHINGTON, D.C. 1883-) 1987, 237(4820), 1346-9<br>* Abstract * | 1 | TECHNICAL FIELDS SEARCHED (Int. Cl.4)<br><br>C 07 K 7/00 |

The present search report has been drawn up for all claims

| Place of search | Date of completion of the search | Examiner |
|---|---|---|
| THE HAGUE | 24-06-1988 | RAJIC M. |

EPO FORM 1503 03.82 (P0401)